# EUROPEAN PATENT SPECIFICATION

(11) **EP 4 498 945 B1**
(45) Date of publication and mention of the grant of the patent: **29.04.2026**
(21) Application number: 23719077.2
(22) Date of filing: 30.03.2023
(51) Int. Cl.: A61B 17/32, A61B 17/29, A61B 17/00, A61B 17/28, A61B 17/22

(54) **ARTICULATING ULTRASONIC SURGICAL INSTRUMENTS HAVING DISTALLY POSITIONED TRANSDUCERS**
GELENKIGE CHIRURGISCHE ULTRASCHALLINSTRUMENTE MIT DISTAL POSITIONIERTEN WANDLERN
INSTRUMENTS CHIRURGICAUX À ULTRASONS ARTICULÉS AYANT DES TRANSDUCTEURS POSITIONNÉS DE MANIÈRE DISTALE

(30) Priority: 30.03.2022 US 202263325195 P
(43) Date of publication of application: 05.02.2025
(73) Proprietor: Covidien LP, Mansfield, MA 02048 (US)
(72) Inventor: COWLEY, Matthew S., Boulder, Colorado 80301 (US); FAGAN, James R., Boulder, Colorado 80301 (US); NEIL, Adam J., Provo, Utah 84604 (US); HAMILTON, Grant J., Highlands Ranch, Utah 80129 (US); BLEDSOE, Jason C., Alpine, Utah 84004 (US); HOWES, Nolan H., West Haven, Utah 84401 (US); BOHN, Wesley S., Ogden, Utah 84403 (US)
(74) Representative: Zimmermann & Partner Patentanwälte mbB
(86) International application number: PCT/IB2023/053175
(87) International publication number: WO 2023/187698

(56) References cited:
- EP-A1- 3 170 462
- EP-A1- 3 714 815
- WO-A1-2021/242656
- US-A1- 2003 225 332
- US-A1- 2014 005 702

## Description

### FIELD

This disclosure relates to surgical instruments and systems and, more particularly, to articulating ultrasonic surgical instruments having distally positioned transducers such as for use in surgical robotic systems.

### BACKGROUND

Ultrasonic surgical instruments and systems utilize ultrasonic energy, i.e., ultrasonic vibrations, to treat tissue. More specifically, a typical ultrasonic surgical instrument or system includes a transducer configured to produce mechanical vibration energy at ultrasonic frequencies that is transmitted along a waveguide to an ultrasonic end effector configured to treat, e.g., seal and transect, tissue.

Some ultrasonic surgical instruments and systems incorporate rotation features, thus enabling rotation of the end effector assembly to a desired orientation within a surgical site. However, the ability to manipulate the end effector assembly within the surgical site via rotation alone is limited.

Adding articulation capability to an ultrasonic surgical instrument increases the positions and orientations the end effector assembly can achieve within a surgical site. However, with the addition of articulation capability comes the challenges of routing mechanical actuation signals, power signals, control signals, and/or mechanical vibration energy to the end effector assembly.
WO2021/242656A1 & EP3170462A1 describe articulating ultrasonic surgical instruments and systems.

### SUMMARY

The invention is defined by the appended independent claims. Optional features are set out in the appended dependent claims.

As used herein, the term "distal" refers to the portion that is being described which is farther from an operator (whether a human surgeon or a surgical robot), while the term "proximal" refers to the portion that is being described which is closer to the operator. Terms including "generally," "about," "substantially," and the like, as utilized herein, are meant to encompass variations, e.g., manufacturing tolerances, material tolerances, use and environmental tolerances, measurement variations, design variations, and/or other variations, up to and including plus or minus 10 percent. To the extent consistent, any of the aspects described herein may be used in conjunction with any or all of the other aspects described herein.

Provided in accordance with aspects of this disclosure is an ultrasonic surgical instrument including a body, an ultrasonic blade extending distally from the body, a jaw member, a jaw open cable, and a jaw close cable. The jaw member includes a rigid structural frame and a compliant jaw liner. The rigid structural frame includes a bifurcated proximal portion and an elongated distal portion. The bifurcated proximal portion includes first and second jaw flags pivotably coupled to the body of the ultrasonic blade to enable pivoting of the jaw member relative to the ultrasonic blade between a spaced-apart position and an approximated position. The compliant jaw liner is secured to the elongated distal portion of the rigid structural frame and configured to oppose the ultrasonic blade in the approximated position of the jaw member. The jaw open cable is coupled to the first jaw flag at a first location and routed along a first path extending about a portion of an outer edge of the first jaw flag. The jaw close cable is coupled to the second jaw flag at a second location and routed along a second path extending about a portion of an outer edge of the second jaw flag. The first and second paths are respectively configured such that proximal pulling of the jaw open cable pivots the jaw member towards the spaced-apart position and such that proximal pulling of the jaw close cable pivots the jaw member towards the approximated position.

In an aspect of this disclosure, the ultrasonic surgical instrument further includes an ultrasonic transducer disposed within the body and an ultrasonic horn coupling the ultrasonic transducer with the ultrasonic blade. In such aspects, the ultrasonic horn may cooperate with the body to sealingly enclose the ultrasonic transducer within a proximal casing of the body. The ultrasonic horn may include, in aspects, an annular flange that is secured between a distal collar of the body and the proximal casing of the body to sealingly enclose the ultrasonic transducer within the proximal casing of the body.

In another aspect of this disclosure, the first and second jaw flags include respective first and second pivot bosses defining a pivot axis and configured for receipt with first and second pivot apertures, respectively, defined through the body on opposite sides of the ultrasonic blade.

In still another aspect of this disclosure, the jaw open cable includes a first ferrule engaged at a distal end thereof. The first ferrule is captured within a first capture recess of the first jaw flag to thereby couple the jaw open cable to the first jaw flag. Alternatively or additionally, the jaw close cable includes a second ferrule engaged at a distal end thereof. The second ferrule is captured within a second capture recess of the second jaw flag to thereby couple the jaw close cable to the second jaw flag.

In yet another aspect of this disclosure, the first and second capture recesses are offset differently from one another relative to a jaw member pivot axis.

In still yet another aspect of this disclosure, the portion of the outer edge of the first jaw flag defines a first cable guide channel configured to at least partially receive the jaw open cable and the portion of the outer edge of the second jaw flag defines a second cable guide channel configured to at least partially receive the jaw close cable.

In another aspect of this disclosure, the body defines first and second slots and first and second guide channels configured to guide the jaw open and close cables proximally from the jaw member.

In another aspect of this disclosure, the body defines a proximal extension configured to couple the body to an articulation joint.

In yet another aspect of this disclosure, the body includes a first distal collar part and a second distal collar part. The first and second distal collar parts are configured to engage one another to define first and second pivot apertures configured to receive first and second pivot bosses of the first and second jaw flags, respectively, to pivotably couple the jaw member to the body.

In still another aspect of this disclosure, the body includes a distal collar defining first and second openings including first and second mouths in communication with the first and second openings, respectively. The first and second openings are configured to receive first and second pivot bosses of the first and second jaw flags through the respective first and second mouths and into the first and second openings, respectively. In such aspects, first and second fillers may be engaged within the first and second mouths, respectively, thereby enclosing the first and second openings and capturing the first and second pivot bosses, respectively, therein.

Another ultrasonic surgical instrument provided in accordance with this disclosure includes a housing, a proximal shaft, an end effector assembly, an articulating section coupling the proximal shaft and the end effector assembly with one another, a jaw open cable, and a jaw close cable. The end effector assembly includes a body housing an ultrasonic transducer therein, an ultrasonic blade coupled to the ultrasonic transducer and extending distally from the body, and a jaw member including first and second jaw flags pivotably coupled to the body to enable pivoting of the jaw member relative to the ultrasonic blade between a spaced-apart position and an approximated position. The jaw open cable is coupled to and routed about the first jaw flag in a first manner such that proximal pulling of the jaw open cable pivots the jaw member towards the spaced-apart position. The jaw close cable is coupled to and routed about the second jaw flag in a second, different manner such that proximal pulling of the jaw close cable pivots the jaw member towards the approximated position.

In an aspect of this disclosure, the jaw open and close cables are routed from the first and second jaw flags, respectively, proximally along the body and through the articulating section and proximal shaft into the housing wherein the jaw open and close cables are operably coupled to a jaw drive sub-assembly.

In another aspect of this disclosure, the housing is configured to releasably engage a surgical robotic system configured to provide at least one input to the jaw drive sub-assembly to actuate the jaw open and close cables.

In yet another aspect of this disclosure, the end effector assembly further includes an ultrasonic horn coupling the ultrasonic transducer with the ultrasonic blade. In such aspects, the ultrasonic horn may cooperate with the body to sealingly enclose the ultrasonic transducer within a proximal casing of the body.

In still another aspect of this disclosure, the first and second jaw flags include respective first and second pivot bosses configured for receipt with first and second pivot apertures, respectively, defined through the body on opposite sides of the ultrasonic blade.

In still yet another aspect of this disclosure, the first jaw flag includes a first capture recess defined therein and a first cable guide channel extending from the first capture recess about a portion of an outer edge of the first jaw flag. The jaw open cable includes a distal end fixed within the first capture recess. The jaw open cable extends from the fixed distal end at least partially within the first cable guide. Additionally or alternatively, the second jaw flag includes a second capture recess defined therein and a second cable guide channel extending from the second capture recess about a portion of an outer edge of the second jaw flag. The jaw close cable includes a distal end fixed within the second capture recess. The jaw close cable extends from the fixed distal end at least partially within the second cable guide.

In another aspect of this disclosure, the first and second jaw flags are pivotably coupled to the body on the opposite sides of the ultrasonic blade via first and second pivot bosses extending from the first and second jaw flags through first and second apertures, respectively, defined within the body. The jaw open cable extends at least partially within the first cable guide on a first side of a pivot axis defined through the first and second pivot bosses and the jaw close cable extends at least partially within the second cable guide on a second, opposite side of the pivot axis.

The details of one or more aspects of this disclosure are set forth in the accompanying drawings and the description below. Other features, objects, and advantages of the techniques described in this disclosure will be apparent from the description and drawings, and from the claims.

### BRIEF DESCRIPTION OF DRAWINGS

Various aspects and features of this disclosure are described hereinbelow with reference to the drawings wherein like numerals designate identical or corresponding elements in each of the several views.
FIG. 1 is a schematic illustration of a surgical robotic system including a control tower, a console, and one or more surgical robotic arms according to aspects of this disclosure;
FIG. 2 is a perspective view of a surgical robotic arm of the surgical robotic system of FIG. 1 according to aspects of this disclosure;
FIG. 3 is a perspective view of a setup arm with the surgical robotic arm of the surgical robotic system of FIG. 1 according to aspects of this disclosure;
FIG. 4 is a schematic diagram of a computer architecture of the surgical robotic system of FIG. 1 according to aspects of this disclosure;
FIG. 5 is a perspective view of a proximal portion of an ultrasonic surgical instrument provided in accordance with this disclosure configured for mounting on a robotic arm of a surgical robotic system such as the surgical robotic system of FIG. 1;
FIGS. 6A and 6B are perspective views of a distal portion of the ultrasonic surgical instrument of FIG. 5 disposed in aligned and articulated positions, respectively;
FIG. 7 is a rear perspective view of the proximal portion of the surgical instrument of FIG. 5 with portions removed;
FIG. 8 is an enlarged, front perspective view of an end effector assembly of the ultrasonic surgical instrument of FIG. 5;
FIGS. 9A and 9B are longitudinal cross-sectional and side views, respectively, of the end effector assembly of FIG. 8;
FIG. 10 is a bottom perspective view of the end effector assembly of FIG. 8;
FIG. 11 is a bottom, rear perspective view of a proximal portion of the end effector assembly of FIG. 8 with portions removed;
FIG. 12 is a front perspective view of a one-piece collar configured for use with the end effector assembly of FIG. 8 or any other suitable end effector assembly;
FIGS. 13A and 13B are front and rear perspective views, respectively, of a rigid structural frame of a jaw member of the end effector assembly of FIG. 8; and
FIG. 14 is a perspective view of the end effector assembly of FIG. 8 including jaw open and close cables operably coupled thereto.

### DETAILED DESCRIPTION

This disclosure provides articulating ultrasonic surgical instruments having distally positioned transducers. As described in detail below, the articulating ultrasonic surgical instruments of this disclosure are configured for use with a surgical robotic system, which may include, for example, a surgical console, a control tower, and one or more movable carts having a surgical robotic arm coupled to a setup arm. The surgical console receives user inputs through one or more interface devices, which are interpreted by the control tower as movement commands for moving the surgical robotic arm. The surgical robotic arm includes a controller, which is configured to process the movement commands and to generate a torque command for activating one or more actuators of the robotic arm, which, in turn, move the robotic arm in response to the movement commands. Those skilled in the art will understand that this disclosure, although described hereinbelow in connection with surgical robotic systems, may also be adapted for use with handheld articulating ultrasonic surgical instruments such as, for example, articulating endoscopic ultrasonic surgical instruments and/or articulating open ultrasonic surgical instruments.

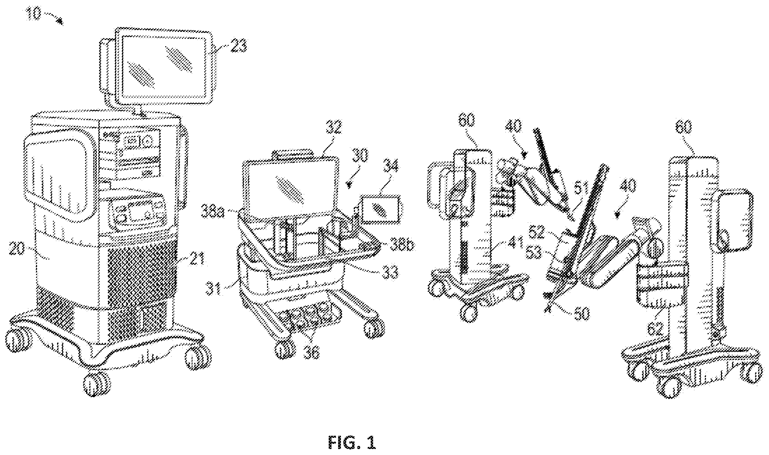

With reference to FIG. 1, a surgical robotic system 10 includes a control tower 20, which is connected to components of the surgical robotic system 10 including a surgical console 30 and one or more robotic arms 40. Each of the robotic arms 40 includes a surgical instrument 50 removably coupled thereto. Each of the robotic arms 40 is also coupled to a movable cart 60.

The one or more surgical instruments 50 may be configured for use during minimally invasive surgical procedures and/or open surgical procedures. In aspects, one of the surgical instruments 50 may be an endoscope, such as an endoscopic camera 51, configured to provide a video feed for the clinician. In further aspects, one of the surgical instruments 50 may be an energy based surgical instrument such as, for example, an electrosurgical forceps or ultrasonic sealing and dissection instrument configured to seal tissue by grasping tissue between opposing structures and applying electrosurgical energy or ultrasonic energy, respectively, thereto. In yet further aspects, one of the surgical instruments 50 may be a surgical stapler including a pair of jaws configured to clamp tissue, deploy a plurality of tissue fasteners, e.g., staples, through the clamped tissue, and/or to cut the stapled tissue. In aspects, one of the surgical instruments 50 is an articulating ultrasonic surgical instrument having a distally positioned transducer in accordance with this disclosure.

One of the robotic arms 40 may include a camera 51 configured to capture video of the surgical site. The surgical console 30 includes a first display 32, which displays a video feed of the surgical site provided by camera 51 of the surgical instrument 50 disposed on the robotic arms 40, and a second display 34, which displays a user interface for controlling the surgical robotic system 10. The first and second displays 32 and 34 are touchscreens allowing for displaying various graphical user inputs.

The surgical console 30 also includes a plurality of user interface devices, such as foot pedals 36 and a pair of handle controllers 38a and 38b which are used by a clinician to remotely control robotic arms 40. The surgical console further includes an armrest 33 used to support clinician's arms while operating the handle controllers 38a and 38b.

The control tower 20 includes a display 23, which may be a touchscreen, and outputs on the graphical user interfaces (GUIs). The control tower 20 also acts as an interface between the surgical console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgical console 30, in such a way that robotic arms 40 and the surgical instrument 50 execute a desired movement sequence in response to input from the foot pedals 36 and the handle controllers 38a and 38b.

Each of the control tower 20, the surgical console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area network, and without limitation as to the full scope of the definition of communication networks as encompassed by this disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth^{®} (an open wireless protocol for exchanging data over short distances, using short length radio waves, from fixed and mobile devices, creating personal area networks (PANs)), ZigBee^{®} (a specification for a suite of high level communication protocols using small, low-power digital radios based on the IEEE 122.15.4-2003 standard for wireless personal area networks (WPANs)).

The computers 21, 31, 41 may include any suitable processor(s) (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor(s) may be any suitable processor(s) (e.g., control circuit(s)) adapted to perform the operations, calculations, and/or set of instructions described in this disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

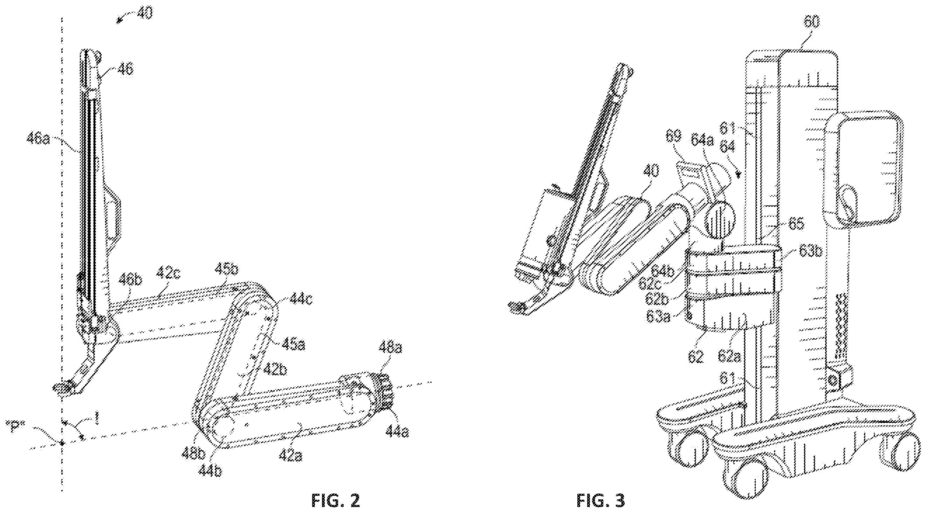

With reference to FIG. 2, each of the robotic arms 40 may include a plurality of links 42a, 42b, 42c, which are interconnected at joints 44a, 44b, 44c, respectively. The joint 44a is configured to secure the robotic arm 40 to the movable cart 60 and defines a first longitudinal axis. With reference to FIG. 3, the movable cart 60 includes a lift 61 and a setup arm 62, which provides a base for mounting of the robotic arm 40. The lift 61 allows for vertical movement of the setup arm 62. The movable cart 60 also includes a display 69 for displaying information pertaining to the robotic arm 40. The setup arm 62 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In aspects, the robotic arm 40 may be coupled to the surgical table (not shown). The setup arm 62 includes controls 65 for adjusting movement of the links 62a, 62b, 62c as well as the lift 61.

The third link 62c includes a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

With reference again to FIG. 2, the robotic arm 40 also includes a holder 46 defining a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components (e.g., end effectors) of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c.

The robotic arm 40 also includes a plurality of manual override buttons 53 disposed on the IDU 52 and the setup arm 62, which may be used in a manual mode. The clinician may press one or the buttons 53 to move the component associated with the button 53.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

The actuator 48b of the joint 44b is coupled to the joint 44c via the belt 45a, and the joint 44c is in turn coupled to the joint 46c via the belt 45b. Joint 44c may include a transfer case coupling the belts 45a and 45b, such that the actuator 48b is configured to rotate each of the links 42b, 42c and the holder 46 relative to each other. More specifically, links 42b, 42c, and the holder 46 are passively coupled to the actuator 48b which enforces rotation about a remote center point "P" which lies at an intersection of the first axis defined by the link 42a and the second axis defined by the holder 46. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle θ. In aspects, some or all of the joints 44a, 44b, 44c may include an actuator to obviate the need for mechanical linkages.

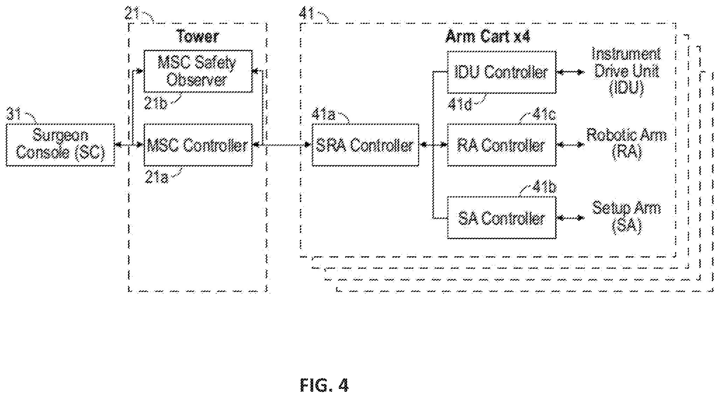

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgical console 30 about the current position and/or orientation of the handle controllers 38a and 38b and the state of the foot pedals 36 and other buttons. The controller 21a processes these input positions to determine desired drive commands for each joint of the robotic arm 40 and/or the IDU 52 and communicates these to the computer 41 of the robotic arm 40. The controller 21a also receives back the actual joint angles and uses this information to determine force feedback commands that are transmitted back to the computer 31 of the surgical console 30 to provide haptic feedback through the handle controllers 38a and 38b. The handle controllers 38a and 38b include one or more haptic feedback vibratory devices that output haptic feedback. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main cart controller 41a, a setup arm controller 41b, a robotic arm controller 41c, and an IDU controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the movable cart 60, the robotic arm 40, and the IDU 52. The main cart controller 41a also communicates actual joint angles back to the controller 21a.

The setup arm controller 41b controls each of joints 63a and 63b, and the rotatable base 64 of the setup arm 62 and calculates desired motor movement commands (e.g., motor torque) for the pitch axis and controls the brakes. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the IDU 52. The IDU controller 41d calculates actual angles based on the motor positions and transmits the actual angles back to the main cart controller 41a.

The robotic arm 40 is controlled as follows. Initially, a pose of the handle controller controlling the robotic arm 40, e.g., the handle controller 38a, is transformed into a desired pose of the robotic arm 40 through a hand eye transform function executed by the controller 21a. The hand eye function, as well as other functions described herein, is/are embodied in software executable by the controller 21a or any other suitable controller described herein. The pose of the handle controller 38a may be embodied as a coordinate position and role-pitch-yaw ("RPY") orientation relative to a coordinate reference frame, which is fixed to the surgical console 30. The desired pose of the instrument 50 is relative to a fixed frame on the robotic arm 40. The pose of the handle controller 38a is then scaled by a scaling function executed by the controller 21a. In aspects, the coordinate position is scaled down and the orientation is scaled up by the scaling function. In addition, the controller 21a also executes a clutching function, which disengages the handle controller 38a from the robotic arm 40. In particular, the controller 21a stops transmitting movement commands from the handle controller 38a to the robotic arm 40 if certain movement limits or other thresholds are exceeded and in essence acts like a virtual clutch mechanism, e.g., limits mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

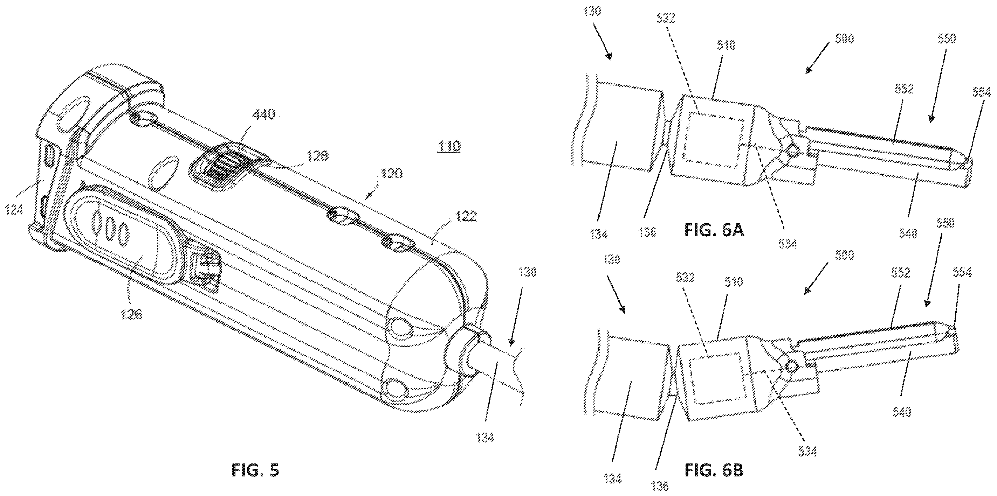

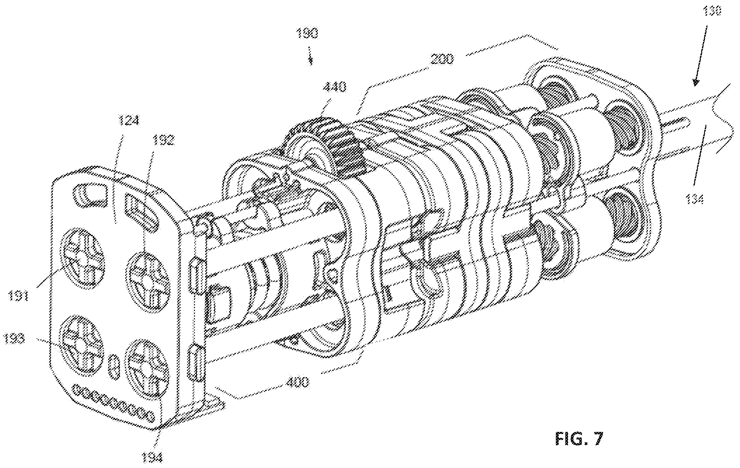

Turning to FIGS. 5-7, a surgical instrument 110 provided in accordance with this disclosure generally includes a housing 120, a shaft 130 extending distally from housing 120, an end effector assembly 500 extending distally from shaft 130, and an actuation assembly 190 (FIG. 7) disposed within housing 120 and operably associated with end effector assembly 500. Instrument 110 is detailed herein as an articulating ultrasonic surgical instrument configured for use with a surgical robotic system, e.g., surgical robotic system 10 (FIG. 1). However, the aspects and features of instrument 110 provided in accordance with this disclosure, detailed below, are equally applicable for use with other suitable surgical instruments and/or in other suitable surgical systems, e.g., motorized, other power-driven systems, and/or manually-actuated surgical systems (including handheld instruments).

Housing 120 of instrument 110 includes a body 122 and a proximal face plate 124 that cooperate to enclose actuation assembly 190 therein. Proximal face plate 124 includes through holes defined therein through which input couplers 191-194 of actuation assembly 190 extend. A pair of latch levers 126 (only one of which is illustrated in FIG. 5) extending outwardly from opposing sides of housing 120 enable releasable engagement of housing 120 with a robotic arm of a surgical robotic system, e.g., surgical robotic system 10 (FIG. 1). A window 128 defined through body 122 of housing 120 permits thumbwheel 440 to extend therethrough to enable manual manipulation of thumbwheel 440 from the exterior of housing 120 to permit manual opening and closing of end effector assembly 500.

Shaft 130 of instrument 110 includes a proximal section 134 and an articulating section 136 disposed between and interconnecting proximal section 134 with end effector assembly 500. Articulating section 136 includes one or more articulating components such as, for example, one or more links, pivots, joints, etc. A plurality of articulation cables (not shown) or other suitable actuators extend through articulating section 136. More specifically, the articulation cables may be operably coupled to end effector assembly 500 at the distal ends thereof and extend proximally through articulating section 136 of shaft 130 and proximal section 134 of shaft 130, and into housing 120, wherein the articulation cables operably couple with an articulation sub-assembly 200 of actuation assembly 190 to enable selective articulation of end effector assembly 500 relative to proximal section 134 and housing 120, e.g., about at least two axes of articulation (yaw and pitch articulation, for example).

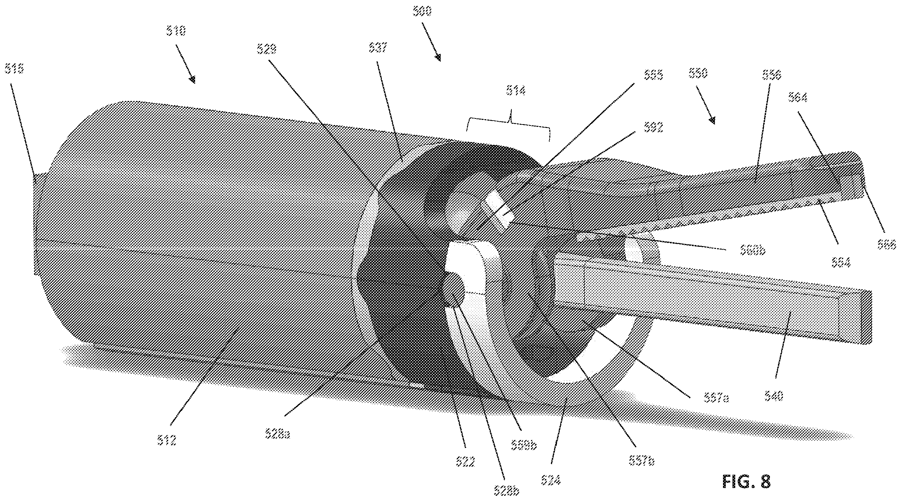

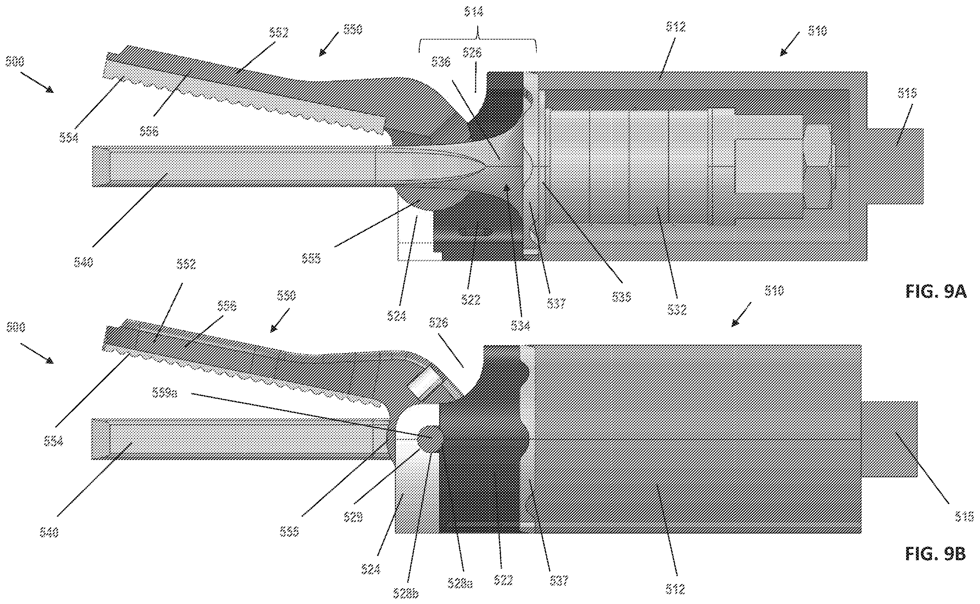

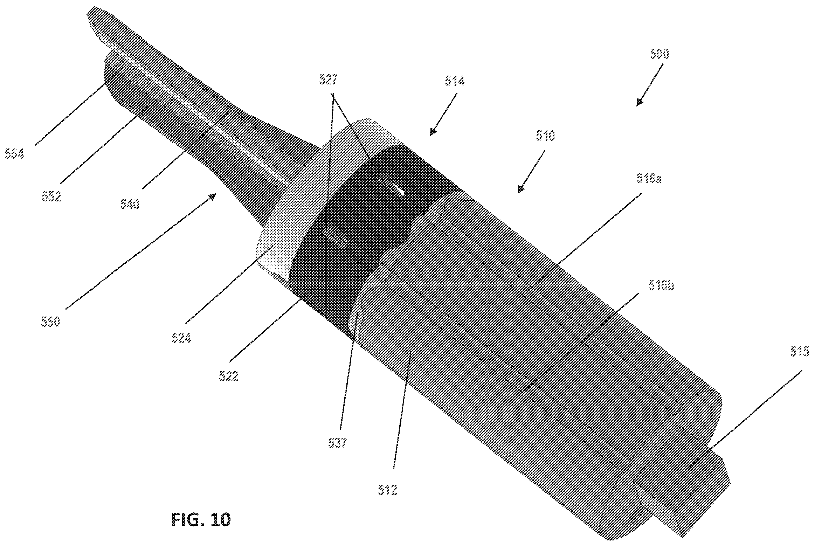

With particular reference to FIGS. 6A and 6B, end effector assembly 500 includes a body 510 retaining ultrasonic transducer 532 therein, an ultrasonic blade 540 operably coupled to ultrasonic transducer 532 via an ultrasonic horn 534 and extending distally from body 510, and a jaw member 550 operably coupled to body 510 to enable pivoting of jaw member 550 relative to ultrasonic blade 540 from a spaced-apart position to an approximated position to clamp tissue between ultrasonic blade 540 and jaw member 550. Jaw member 550 includes a rigid structural frame 552 that is operably coupled to body 510, and a compliant jaw liner 554 that is captured by rigid structural frame 552 and positioned to oppose ultrasonic blade 540 to enable clamping of tissue therebetween. End effector assembly 500 is described in greater detail below with reference to FIGS. 8-14.

Referring again to FIGS. 5-7, actuation assembly 190 is disposed within housing 120 and includes an articulation sub-assembly 200 and a jaw drive sub-assembly 400. Articulation sub-assembly 200 is operably coupled between first and second input couplers 191, 192, respectively, of actuation assembly 190 and the articulation cables such that, upon receipt of appropriate inputs into first and/or second input couplers 191, 192, articulation sub-assembly 200 manipulates the articulation cables to articulate end effector assembly 500 in a desired direction, e.g., to pitch and/or yaw end effector assembly 500.

Jaw drive sub-assembly 400 operably couples third and fourth input couplers 193, 194 of actuation assembly 190 with jaw member 550 such that, upon receipt of appropriate input into third coupler 193, jaw drive sub-assembly 400 pivots jaw member 550 towards the approximated position to clamp tissue between and apply a jaw force within an appropriate jaw force range to tissue clamped between compliant jaw liner 554 of jaw member 550 and ultrasonic blade 540, and such that, upon receipt of appropriate input into fourth input coupler 194, jaw drive sub-assembly 400 pivots jaw member 550 towards the spaced-apart position to release clamped tissue. Alternatively, jaw drive sub-assembly 400 may be configured to receive a single input, e.g., into third input coupler 193 or fourth input coupler 194, to enable both opening and closing jaw member 550, e.g., wherein the input in a first direction closes jaw member 550 and the input in a second, opposite direction opens the jaw member 550. In either configuration, jaw drive sub-assembly 400 may be tuned to provide a jaw clamping force, or jaw clamping force within a jaw clamping force range, to tissue clamped between jaw member 550 and ultrasonic blade 540, such as described in U.S. Patent Application No. 17/071,263, filed on October 15, 2020.
Alternatively, the jaw drive sub-assembly 400 may include a force limiting feature, e.g., a spring, whereby the clamping force applied to tissue clamped between jaw member 550 and ultrasonic blade 540 is limited to a particular jaw clamping force or a jaw clamping force within a jaw clamping force range, such as described in U.S. Patent No. 10,368,898, issued on August 6, 2019.

Actuation assembly 190 is configured to operably interface with a surgical robotic system, e.g., system 10 (FIG. 1), when instrument 110 is mounted on a robotic arm thereof, to enable robotic operation of actuation assembly 190 to provide the above detailed functionality. That is, surgical robotic system 10 (FIG. 1) selectively provides inputs, e.g., rotational inputs to input couplers 191-194 of actuation assembly 190 to articulate end effector assembly 550, clamp tissue between jaw member 550 and ultrasonic blade 540, and release tissue (e.g., sealed and transected tissue) from between jaw member 550 and ultrasonic blade 540. However, as noted above, it is also contemplated that actuation assembly 190 be configured to interface with any other suitable surgical systems, e.g., a manual surgical handle, a powered surgical handle, etc.

Turing to FIGS. 8-14, end effector assembly 500 is described in more detail. End effector assembly 500 may be utilized with surgical instrument 110 (FIGS. 5-7) or any other suitable surgical instrument and, as noted above, generally includes body 510 retaining ultrasonic transducer 532 therein, an ultrasonic horn 534 coupled to and extending distally from ultrasonic transducer 534, an ultrasonic blade 540 coupled to and extending distally from ultrasonic horn 534 and body 510, and jaw member 550 operably coupled to body 510 to enable pivoting of jaw member 550 relative to ultrasonic blade 540 between the spaced-apart position and the approximated position.

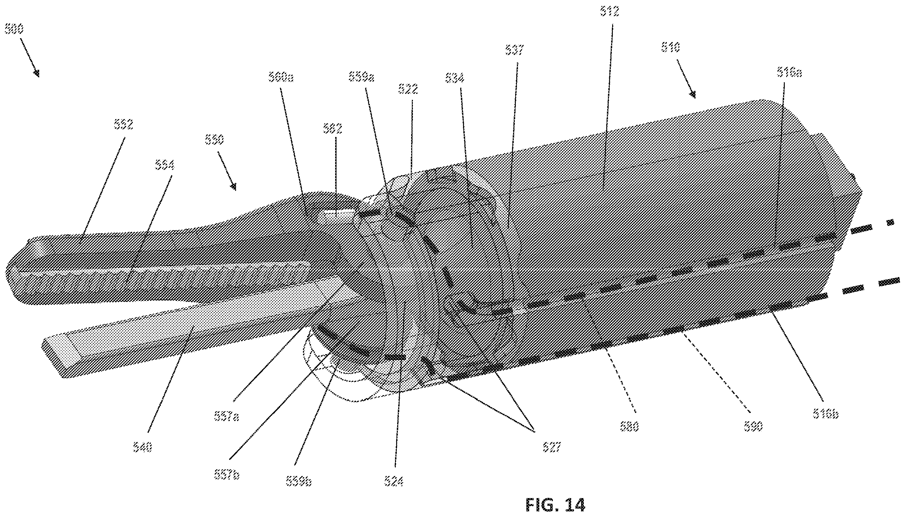

Referring to FIGS. 8-11, body 510 of end effector assembly 500 includes a proximal casing 512 and a distal collar 514 that cooperate to enclose and secure ultrasonic transducer 532 therein. Proximal casing 512 includes a proximal extension 515 configured to facilitate mechanical engagement of body 510 with articulating section 136 of shaft 130 of surgical instrument 110 (see FIGS. 5-6B). Proximal casing 512 further includes first and second cable guide channels 516a, 516b configured to guide jaw open and close cables 580, 590 (see FIG. 14) from jaw member 550 proximally along an exterior of proximal casing 512 into and through articulating section 136 of shaft 130 of surgical instrument 110 (see FIGS. 5-6B) for connecting to jaw drive sub-assembly 400 of actuation assembly 190 (FIG. 7). Proximal casing 512 also includes proximal apertures 518 configured to receive electrode leads 570 (only one of which is shown) to enable connection of electrode leads 570 to ultrasonic transducer 532 within proximal casing 512. In aspects, proximal apertures 518 are sealed about electrode leads 570 to inhibit fluid ingress therethrough and into proximal casing 512.

Distal collar 514 is formed from first and second collar parts 522, 524, respectively. First collar part 522 is configured to cooperate with an open distal end of proximal casing 512 to sandwich annular flange 537 of ultrasonic horn 534 therebetween. To this end, first collar part 522 and annular flange 537 may define complementary engaging features, e.g., corresponding recesses and protrusions, to facilitate proper securement therebetween. Likewise, the open distal end of proximal casing 512 and annular flange 537 may define complementary engaging features, e.g., corresponding recesses and protrusions, to facilitate proper securement therebetween. The open distal end of proximal casing 512 is secured to annular flange 537 on a proximal side of annular flange 537, e.g., via welding, and first collar part 522 is secured to annular flange 537 on a distal side of annular flange 537, e.g., via welding, to secure proximal casing 512, annular flange 537, and first collar part 522 to one another, thereby securing and sealingly enclosing ultrasonic transducer 532 within proximal casing 512.

Proximal casing 512 may additionally or alternatively be configured to engage ultrasonic horn 534 and sealingly enclosing ultrasonic transducer 532 therein similarly as detailed in Patent Application Publication Nos. US 2019/0231385, US 2021/0369295, and/or WO 2021/006984.

Continuing with reference to FIGS. 8-11, first collar part 522 further includes a distal cut-out 526, a pair of slots 527 defined on an underside thereof, and opposing lateral recesses 528a defined within a distally facing surface of first collar part 522. Slots 527 are configured to facilitate routing of jaw open and close cables 580, 590 from within first collar part 522, wherein jaw open and close cables 580, 590 operably engage jaw member 550, to cable guide channels 516a, 516b on the exterior of proximal casing 512.

Second collar part 524 defines a substantially U-shaped configuration that, together with distal cut-out 526 of first collar 522, provides sufficient clearance for pivoting of jaw member 550 relative to body 510. Second collar part 524 further includes opposing lateral recesses 528b defined within a proximally facing surface thereof. Upon securing, e.g., welding, first and second collar parts 522, 524 with one another, opposing lateral recesses 528a, 528b cooperate to define opposing apertures 529 that capture pivot bosses 559a, 559b of jaw member 550 to pivotably couple jaw member 550 to body 510.

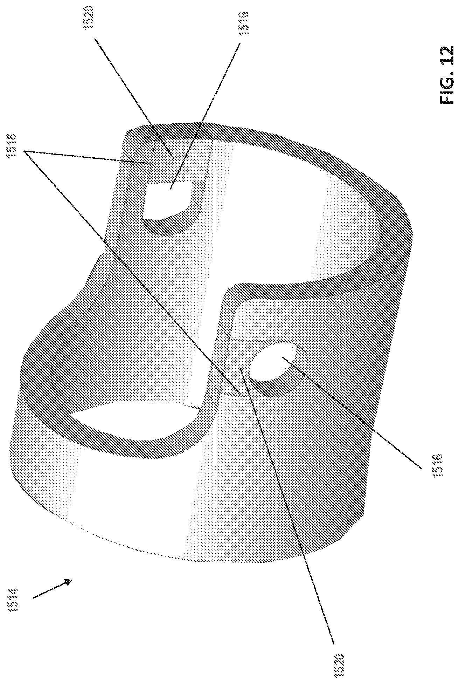

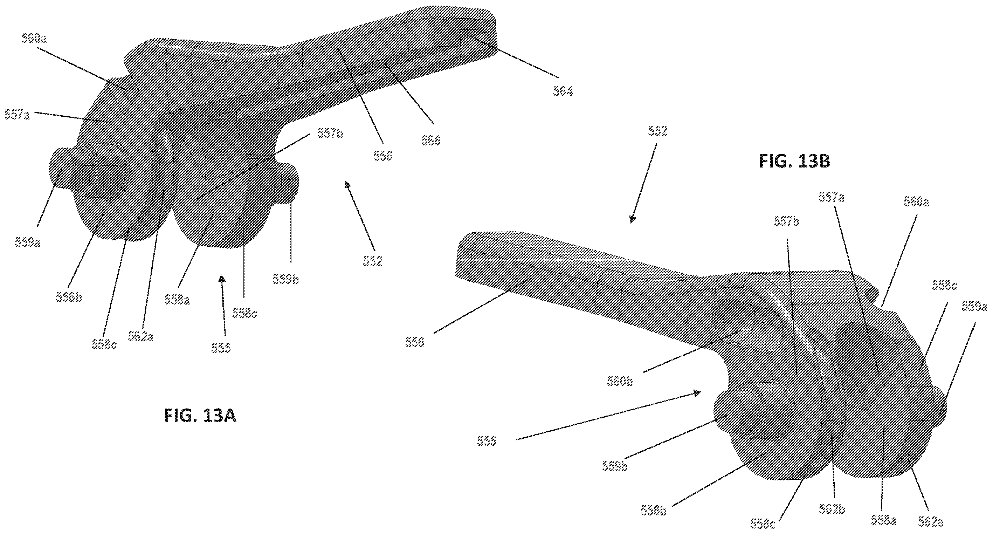

Referring momentarily to FIG. 12, as an alternative to distal collar 514 being formed from first and second collar parts 522, 524 that cooperate to define opposing apertures 529 to capture pivot bosses 559a, 559b of jaw member 550 to pivotably couple jaw member 550 to body 510 (see FIGS. 8-11), a unitary distal collar 1514 may be provided. Unitary distal collar 1514 defines opposed openings 1516 having mouths 1518 providing access thereto, e.g., for sliding pivot bosses 559a, 559b of jaw member 550 (FIGS. 13A and 13B) into openings 1516. With pivot bosses 559a, 559b of jaw member 550 (FIGS. 13A and 13B) received within openings 1516, mouths 1518 may be closed off, e.g., via welding fillers 1520 within the gaps defined by mouths 1518, thus enclosing openings 1516 and capturing pivot bosses 559a, 559b of jaw member 550 (FIGS. 13A and 13B) within openings 1516 to thereby pivotably couple jaw member 550 (FIGS. 13A and 13B) to distal collar 1514.

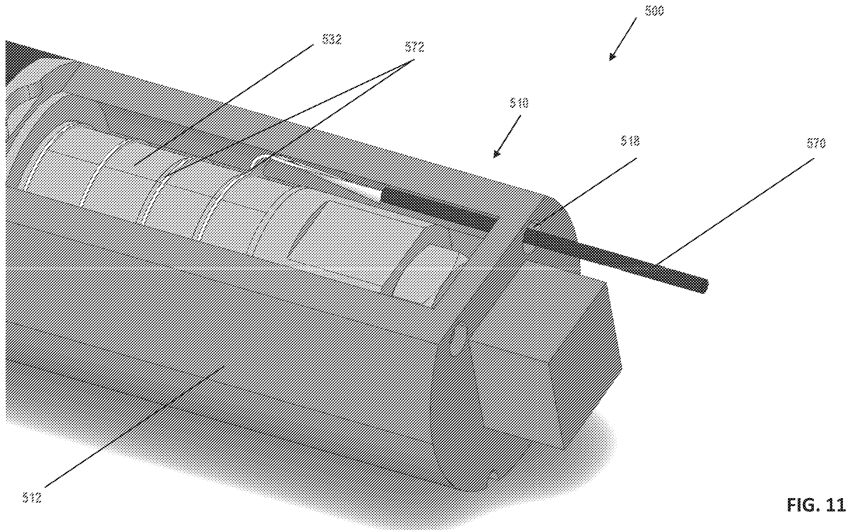

With reference again to FIGS. 8-11, ultrasonic transducer 532 may include a stack of piezoelectric elements secured, under pre-compression between proximal and distal end masses or a proximal end mass and ultrasonic horn 534 with electrodes 572 electrically coupled between piezoelectric elements of the stack of piezoelectric elements to enable energization thereof to produce ultrasonic energy. However, other suitable ultrasonic transducer configurations, including plural transducers and/or non-linear transducers are also contemplated. Electrical lead wires 570 (only one of which is shown in FIG. 11) extend proximally from end effector assembly 500, e.g., through shaft 130 and housing 120 (FIG. 5), to connect the electrodes 572 of ultrasonic transducer 532 with an ultrasonic generator (not shown) to enable an electrical drive signal generated by the ultrasonic generator to be imparted to the stack of piezoelectric elements of ultrasonic transducer 532 to energize the stack of piezoelectric elements to produce ultrasonic energy for transmission to ultrasonic blade 540 via ultrasonic horn 534 to treat tissue, e.g., seal, transect, dissect, score, perform an otomy, or otherwise treat tissue.

Ultrasonic horn 534 is engaged to the stack of piezoelectric elements of ultrasonic transducer 532 and extends distally therefrom. Ultrasonic horn 534 includes a base 535 disposed within proximal casing 512 and a nose 536 extending distally from base 535 externally of proximal casing 512. Annular flange 537 is disposed between base 535 and nose 536. As noted above, annular flange 537 is sealed, e.g., via welding, between distal collar 514 and proximal casing 512 to thereby sealingly enclose ultrasonic transducer 532 within proximal casing 512. In aspects, annular flange 537 is disposed at or near a nodal point along ultrasonic horn 534. Nose 536 of ultrasonic horn 534 may define a distal threaded female receiver configured to enable releasable threaded engagement of ultrasonic blade 540 therewith or may be unitarily formed with ultrasonic blade 540 as a single component.

Ultrasonic blade 540 extends distally from ultrasonic horn 534 and distally from distal collar 514 of body 510. Ultrasonic blade 540 may define a curved configuration wherein the directions of movement of jaw member 550 between the spaced-apart and approximated positions are perpendicular to the direction of curvature of ultrasonic blade 540. However, it is also contemplated that ultrasonic blade 540 define a straight configuration or that ultrasonic blade 540 additionally or alternatively curve towards or away from jaw member 550; that is, where the directions of movement of jaw member 550 between the spaced-apart and approximated positions are coplanar or parallel to the direction of curvature of ultrasonic blade 540. Multiple curvatures of ultrasonic blade 540 (in the same or different directions) and/or combinations of curved and linear portions of ultrasonic blade 540 are also contemplated. Likewise, some portions or surfaces of ultrasonic blade 540 may be curved while others are not curved. Ultrasonic blade 540 may additionally or alternatively taper in width (a dimension perpendicular to the directions of movement of jaw member 550 in a proximal-to-distal direction and/or in height (a dimension parallel or coplanar with the directions of movement of jaw member 550) in a proximal-to-distal direction. Other configurations are also contemplated.

Referring also to FIGS. 13A-14, jaw member 550 of end effector assembly 500, as noted above, includes rigid structural frame 552 and compliant jaw liner 554. Rigid structural frame 552 includes a bifurcated proximal portion 555 (e.g., to receive ultrasound blade 540 therebetween) and an elongated distal portion 556 extending distally from bifurcated proximal portion 555. Bifurcated proximal portion 555 includes first and second spaced-apart jaw flags 557a, 557b each defining an inner face 558a, an outer face 558b, and an edge 558c between the inner and outer faces 558a, 558b, respectively. Pivot bosses 559a, 559b are aligned with one another (thereby defining a pivot axis), extend outwardly from outer faces 558b of flags 557a, 557b, and are configured for receipt within opposing apertures 529 of body 510 to thereby pivotably couple jaw member 550 with body 510. Each jaw flag 557a, 557b further defines a capture recess 560a, 560b therein and includes a cable guide channel 562a, 562b extending about at least a portion of the corresponding edge 558c thereof.

At least a portion of each edge 558c is arcuate such that at least a portion of each cable guide channel 562a, 562b is also arcuate. The arcuate portions of edges 558c of jaw flags 557a, 557b and, thus, cable guide channels 562a, 562b thereof, may define constant radii of curvature (that are the same as or different from one another) centered about or off-center relative to pivot bosses 559a, 559b. Alternatively, at least a portion of each edge 558c may define at least a portion of an oval (wherein the ovals of jaw flags 557a, 557b are the same or different from one another) such that cable guide channels 562a, 562b extend along portions of ovals.

Each capture recess 560a, 560b is disposed at an end of the corresponding cable guide channel 562a, 562b. Capture recesses 560a, 560b and cable guide channels 562a, 562b are positioned differently on jaw flags 557a, 557b such that, with respect to jaw flag 557a, cable guide channel 562a extends from capture recess 560a about at least a portion of jaw flag 557a on a first side, e.g., above, pivot bosses 559a, 559b while, with respect to the other jaw flag 557b, cable guide channel 562b extends from capture recess 560b about at least a portion of jaw flag 557b on a second, opposite side, e.g., below, pivot bosses 559a, 559b. Capture recesses 560a, 560b and cable guide channels 562a, 562b are configured to receive respective jaw open and close cables 580, 590 to enable pivoting of jaw member 550 towards and away from ultrasonic blade 540, respectively, as detailed below.

Continuing with reference to FIGS. 8-11 and 13A-14, as noted above, an elongated distal portion 556 of rigid structural frame 552 of jaw member 550 extends distally from bifurcated proximal portion 555 of rigid structural frame 552. Elongated distal portion 556 may be curved, linear, or otherwise configured complementary to the configuration of ultrasonic blade 540. Elongated distal portion 556 includes a longitudinally extending channel 564 defined within a blade-facing surface 566 thereof and extending along at least a portion of a length of elongated distal portion 556. Longitudinally extending channel 564 may define a T-shaped, L-shaped, or other suitable keyed configuration to facilitate drop-in or slidable engagement and capture of compliant jaw liner 554 within longitudinally extending channel 564 of elongated distal portion 556 of rigid structural frame 552. With compliant jaw liner 554 captured within longitudinally extending channel 564, compliant jaw liner 554 protrudes from blade-facing surface 566 towards ultrasonic blade 540 such that, in the approximated position of jaw member 550, compliant jaw liner 554 is positioned to oppose ultrasonic blade 540 to enable clamping of tissue therebetween. Compliant jaw liner 554 also inhibits contact between ultrasonic blade 540 and rigid structural frame 552.

Jaw open and close cables 580, 590 (FIG. 14) include ferrules 582, 592 (see also FIG. 8) engaged thereon at distal ends thereof. Ferrules 582, 592 are received within capture recesses 560a, 560b (see also FIG. 8) of jaw flags 557a, 557b, respectively, to thereby fix the distal ends of jaw open and close cables 580, 590 relative to jaw member 550. Jaw open and close cables 580, 590 extend proximally from capture recesses 560a, 560b through cable guide channels 562a, 562b (with jaw open cable 580 extending from capture recess 560a about at least a portion of jaw flag 557a on the first side, e.g., above, pivot bosses 559a, 559b and jaw close cable 590 extending from capture recess 560b about at least a portion of jaw flag 557b on the second, opposite side, e.g., below, pivot bosses 559a, 559b), through slots 527 of body 510 and at least partially within cable guide channels 516a, 516b on the exterior of proximal casing 512. Jaw open and close cables 580, 590 are routed proximally from cable guide channels 516a, 516b, respectively, into and through articulating section 136 of shaft 130 of surgical instrument 110 (see FIGS. 5-6B) for connecting to jaw drive sub-assembly 400 of actuation assembly 190 (see FIG. 7). In aspects, jaw open and close cables 580, 590 are associated with separate inputs to jaw drive sub-assembly 400 of actuation assembly 190 (FIG. 7), e.g., a first input to input coupler 194 (FIG. 7) for actuating jaw open cable 580 to open jaw member 550 relative to ultrasonic blade 540 and a second input to input coupler 193 (FIG. 7) for actuating jaw close cable 590 to close jaw member 550 relative to ultrasonic blade 540. In other aspects, jaw open and close cables 580, 590 are coupled to one another (e.g., at end effector assembly 500, within shaft 130 (FIG. 5), or within housing 120 (FIG. 5) and associated with a single input to jaw drive sub-assembly 400 of actuation assembly 190 (FIG. 7), e.g., wherein an input to input coupler 193 (FIG. 7) in a first direction actuates jaw open cable 580 to open jaw member 550 relative to ultrasonic blade 540 and an input to input coupler 193 (FIG. 7) in a second, opposite direction actuates jaw close cable 590 to close jaw member 550 relative to ultrasonic blade 540.

More specifically, in order to pivot jaw member 550 towards the spaced-apart position, jaw drive sub-assembly 400 of actuation assembly 190 (FIG. 7) is actuated to pull jaw open cable 580 proximally. As a result of the above-detailed configuration of jaw member 550, proximal pulling of jaw open cable 580 provides a pulling force at capture recesses 560a from the first side, e.g., above, pivot bosses 559a, 559b, thereby urging jaw member 550 to pivot away from ultrasonic blade 540. On the other hand, in order to pivot jaw member 550 towards the approximated position, jaw drive sub-assembly 400 of actuation assembly 190 (FIG. 7) is actuated to pull jaw close cable 590 proximally. As a result of the above-detailed configuration of jaw member 550, proximal pulling of jaw close cable 590 provides a pulling force at capture recesses 560b from the second side, e.g., below, pivot bosses 559a, 559b, thereby urging jaw member 550 to pivot towards ultrasonic blade 540.

Jaw flags 557a, 557b, cable guide channels 562a, 562b, and/or capture recesses 560a, 560b may be configured, e.g., relative to pivot bosses 559a, 559b, to provide a desired jaw closure and/or jaw opening configuration., e.g., by varying the arcuate shapes of cable guide channels 562a, 562b along their lengths and/or relative to one another, as detailed above. For example, the above-noted components may be configured such that jaw member 550 is pivoted a greater amount in response to pulling of jaw close cable 590 during initial jaw closure and a smaller amount in response to the same pulling of jaw close cable 590 as jaw member 550 approaches the approximated position, although the opposite is also contemplated. As another example, the above-noted components may be configured differently on one side as compared the other such that jaw member 550 is pivoted a smaller amount (in magnitude) towards the approximated position in response to pulling of jaw close cable 590 and is pivoted a greater amount (in magnitude) towards the spaced-apart position in response to the same pulling of jaw open cable 580, although the opposite is also contemplated.

Although this disclosure is detailed with respect to an ultrasonic surgical instrument including a distally positioned transducer, this disclosure is equally applicable to other suitable surgical instruments. For example and without limitation, the transducer, ultrasonic horn, and ultrasonic blade may be replaced with energy-generating electrodes and a fixed jaw member configured for positioning opposite the movable jaw member. In such configurations, the proximal body of the end effector assembly may house power, energy-generating, and/or control electronics to operate an energy-based component associated with the fixed jaw member and/or the movable jaw member, e.g., either or both including an RF electrode for monopolar or bipolar tissue treatment, a thermal cutting element configured to thermally treat tissue, a microwave probe, etc.

It will be understood that various modifications may be made to the aspects and features disclosed herein. Therefore, the above description should not be construed as limiting, but merely as exemplifications of various configurations. Those skilled in the art will envision other modifications within the scope of the claims appended hereto.

## Claims

1. An ultrasonic surgical instrument (110), comprising:
an end effector assembly (500) including a body retaining an ultrasonic blade (540);
the ultrasonic blade (540) extending distally from the body (510);
a jaw member (550), including:
a rigid structural frame (552) including a bifurcated proximal portion (555) and an elongated distal portion (556), the bifurcated proximal portion (555) including first and second jaw flags (557a, 557b) pivotably coupled to the body (510) to enable pivoting of the jaw member (550) relative to the ultrasonic blade (540) between a spaced-apart position and an approximated position; and
a compliant jaw liner (554) secured to the elongated distal portion (556) of the rigid structural frame and configured to oppose the ultrasonic blade (540) in the approximated position of the jaw member;
a jaw open cable (580) coupled to the first jaw flag (557a) at a first location and routed about the pivot axis along a first path extending about a portion of an outer edge of the first jaw flag (557a); and
a jaw close cable (590) coupled to the second jaw flag (557b) at a second location and routed about the pivot axis along a second path extending about a portion of an outer edge of the second jaw flag (557b),
wherein the first and second paths are respectively configured such that proximal pulling of the jaw open cable (580) pivots the jaw member (550) towards the spaced-apart position and such that proximal pulling of the jaw close cable (590) pivots the jaw member (550) towards the approximated position.

2. The ultrasonic surgical instrument (110) according to claim 1, further comprising:
an ultrasonic transducer (532) disposed within the body (510); and
an ultrasonic horn (534) coupling the ultrasonic transducer (532) with the ultrasonic blade (540).

3. The ultrasonic surgical instrument (110) according to claim 2, wherein the ultrasonic horn (534) cooperates with the body (510) to sealingly enclose the ultrasonic transducer within a proximal casing of the body.

4. The ultrasonic surgical instrument (110) according to claim 3, wherein the ultrasonic horn (534) includes an annular flange (537), and wherein the annular flange (537) is secured between a distal collar (514) of the body and the proximal casing of the body to sealingly enclose the ultrasonic transducer (532) within the proximal casing of the body.

5. The ultrasonic surgical instrument (110) according to any of claims 1 to 4, wherein the first and second jaw flags (557a, 557b) include respective first and second pivot bosses (559a, 559b) defining a pivot axis and configured for receipt with first and second pivot apertures (529), respectively, defined through the body on opposite sides of the ultrasonic blade (540).

6. The ultrasonic surgical instrument (110) according to any of claims 1 to 5, wherein:
the jaw open cable (580) includes a first ferrule (582) engaged at a distal end thereof, the first ferrule (582) captured within a first capture recess of the first jaw flag (557a) to thereby couple the jaw open cable (580) to the first jaw flag (557a); and
the jaw close cable (590) includes a second ferrule (592) engaged at a distal end thereof, the second ferrule (592) captured within a second capture recess of the second jaw flag (557b) to thereby couple the jaw close cable (590) to the second jaw flag (557b).

7. The ultrasonic surgical instrument (110) according to claim 6, wherein the first and second capture recesses are offset differently from one another relative to a pivot axis of the jaw member.

8. The ultrasonic surgical instrument (110) according to any of claims 1 to 7, wherein:
the portion of the outer edge of the first jaw flag (557a) defines a first cable guide channel (562a) configured to at least partially receive the jaw open cable (580); and
the portion of the outer edge of the second jaw flag (557b) defines a second cable guide channel (562b) configured to at least partially receive the jaw close cable (590).

9. The ultrasonic surgical instrument (110) according to claim 8, wherein:
the body (510) defines a first slot (527) and the first guide channel configured to guide the jaw open cable (580) proximally from the jaw member; and
the body defines a second slot (527) and the second guide channel configured to guide the jaw close cable (590) proximally from the jaw member.

10. The ultrasonic surgical instrument (110) according to any of claims 1 to 9, wherein the body (510) defines a proximal extension configured to couple the body to an articulation joint.

11. The ultrasonic surgical instrument (110) according to any of claims 1 to 10, wherein the body (510) includes a first distal collar part and a second distal collar part, the first and second distal collar parts configured to engage one another to define first and second pivot apertures configured to receive first and second pivot bosses (559a, 559b) of the first and second jaw flags (557a, 557b), respectively, to pivotably couple the jaw member to the body.

12. The ultrasonic surgical instrument (110) according to any of claims 1 to 11, wherein the body (510) includes a distal collar defining first and second openings and including first and second mouths (1518) in communication with the first and second openings, respectively, the first and second openings configured to receive first and second pivot bosses of the first and second jaw flags (557a, 557b) through the first and second mouths (1518) and into the first and second openings, respectively.

13. The ultrasonic surgical instrument (110) according to claim 12, further comprising first and second fillers (1520) engaged within the first and second mouths (1518), respectively, thereby enclosing the first and second openings and capturing the first and second pivot bosses (559a, 559b), respectively, therein.

## Patentansprüche

1. Chirurgisches Ultraschallinstrument (110), umfassend:
eine Endeffektoranordnung (500), die einen Körper einschließt, der eine Ultraschallklinge (540) hält;
wobei sich die Ultraschallklinge (540) distal von dem Körper (510) erstreckt;
ein Backenelement (550), welches einschließt:
einen starren strukturellen Rahmen (552), der einen gegabelten proximalen Abschnitt (555) und einen länglichen distalen Abschnitt (556) einschließt, wobei der gegabelte proximale Abschnitt (555) erste und zweite Backenfähnchen (557a, 557b) einschließt, die schwenkbar mit dem Körper (510) gekoppelt sind, um Schwenken des Backenelements (550) relativ zu der Ultraschallklinge (540) zwischen einer beabstandeten Position und einer angenäherten Position zu ermöglichen; und
eine nachgiebige Backenauskleidung (554), die an dem länglichen distalen Abschnitt (556) des starren strukturellen Rahmens befestigt ist und dazu ausgelegt ist, der Ultraschallklinge (540) in der angenäherten Position des Backenelements gegenüberzuliegen;
ein Backenöffnungskabel (580), das an einer ersten Stelle mit dem ersten Backenfähnchen (557a) gekoppelt ist und um die Schwenkachse entlang eines ersten Pfades geführt ist, der sich um einen Abschnitt einer Außenkante des ersten Backenfähnchens (557a) erstreckt; und
ein Backenschließkabel (590), das an einer zweiten Stelle mit dem zweiten Backenfähnchen (557b) gekoppelt ist und um die Schwenkachse entlang eines zweiten Pfades geführt ist, der sich um einen Abschnitt einer Außenkante des zweiten Backenfähnchens (557b) erstreckt,
wobei der erste und der zweite Pfad jeweils so ausgelegt sind, dass proximales Ziehen des Backenöffnungskabels (580) das Backenelement (550) in Richtung der beabstandeten Position schwenkt, und so, dass ein proximales Ziehen des Backenschließkabels (590) das Backenelement (550) in Richtung der angenäherten Position schwenkt.

2. Chirurgisches Ultraschallinstrument (110) nach Anspruch 1, ferner umfassend:
einen Ultraschallwandler (532), der innerhalb des Körpers (510) angeordnet ist; und
ein Ultraschallhorn (534), das den Ultraschallwandler (532) mit der Ultraschallklinge (540) koppelt.

3. Chirurgisches Ultraschallinstrument (110) nach Anspruch 2, wobei das Ultraschallhorn (534) mit dem Körper (510) zusammenwirkt, um den Ultraschallwandler innerhalb eines proximalen Gehäuses des Körpers abdichtend zu umschließen.

4. Chirurgisches Ultraschallinstrument (110) nach Anspruch 3, wobei das Ultraschallhorn (534) einen ringförmigen Flansch (537) einschließt, und wobei der ringförmige Flansch (537) zwischen einem distalen Kragen (514) des Körpers und dem proximalen Gehäuse des Körpers befestigt ist, um den Ultraschallwandler (532) innerhalb des proximalen Gehäuses des Körpers abdichtend zu umschließen.

5. Chirurgisches Ultraschallinstrument (110) nach einem der Ansprüche 1 bis 4, wobei das erste und das zweite Backenfähnchen (557a, 557b) jeweilige erste und zweite Schwenkzapfen (559a, 559b) einschließen, die eine Schwenkachse definieren und zur Aufnahme mit ersten bzw. zweiten Schwenköffnungen (529) ausgelegt sind, die durch den Körper auf gegenüberliegenden Seiten der Ultraschallklinge (540) definiert sind.

6. Chirurgisches Ultraschallinstrument (110) nach einem der Ansprüche 1 bis 5, wobei:
das Backenöffnungskabel (580) eine erste Klemmhülse (582) einschließt, die an einem distalen Ende davon in Eingriff steht, wobei die erste Klemmhülse (582) in einer ersten Halteaussparung des ersten Backenfähnchens (557a) gehalten wird, um dadurch das Backenöffnungskabel (580) an das erste Backenfähnchen (557a) zu koppeln; und
das Backenschließkabel (590) eine zweite Klemmhülse (592) einschließt, die an einem distalen Ende davon in Eingriff steht, wobei die zweite Klemmhülse (592) in einer zweiten Halteaussparung des zweiten Backenfähnchen (557b) gehalten wird, um dadurch das Backenschließkabel (590) mit der zweiten Backenfähnchen (557b) zu koppeln.

7. Chirurgisches Ultraschallinstrument (110) nach Anspruch 6, wobei die erste und die zweite Halteaussparung relativ zu einer Schwenkachse des Backenelements unterschiedlich zueinander versetzt sind.

8. Chirurgisches Ultraschallinstrument (110) nach einem der Ansprüche 1 bis 7, wobei:
der Abschnitt der Außenkante des ersten Backenfähnchens (557a) einen ersten Kabelführungskanal (562a) definiert, der dazu ausgelegt ist, das Backenöffnungskabel (580) zumindest teilweise aufzunehmen; und
der Abschnitt der Außenkante des zweiten Backenfähnchens (557b) einen zweiten Kabelführungskanal (562b) definiert, der dazu ausgelegt ist, das Backenschließkabel (590) zumindest teilweise aufzunehmen.

9. Chirurgisches Ultraschallinstrument (110) nach Anspruch 8, wobei:
der Körper (510) einen ersten Schlitz (527) definiert, und der erste Führungskanal dazu ausgelegt ist, das Backenöffnungskabel (580) proximal von dem Backenelement zu führen; und
der Körper einen zweiten Schlitz (527) definiert, und der zweiten Führungskanal dazu ausgelegt ist, das Backenschließkabel (590) proximal von dem Backenelement zu führen.

10. Chirurgisches Ultraschallinstrument (110) nach einem der Ansprüche 1 bis 9, wobei der Körper (510) eine proximale Verlängerung definiert, die dazu ausgelegt ist, den Körper mit einem Knickgelenk zu koppeln.

11. Chirurgisches Ultraschallinstrument (110) nach einem der Ansprüche 1 bis 10, wobei der Körper (510) einen ersten distalen Kragenteil und einen zweiten distalen Kragenteil einschließt, wobei der erste und der zweite distale Kragenteil dazu ausgelegt sind, miteinander in Eingriff zu kommen, um erste und zweite Schwenköffnungen zu definieren, die dazu ausgelegt sind, erste und zweite Schwenkzapfen (559a, 559b) der ersten bzw. zweiten Backenfähnchen (557a, 557b) aufzunehmen, um das Backenelement schwenkbar mit dem Körper zu koppeln.

12. Chirurgisches Ultraschallinstrument (110) nach einem der Ansprüche 1 bis 11, wobei der Körper (510) einen distalen Kragen einschließt, der erste und zweite Öffnungen definiert und erste und zweite Mündungen (1518) einschließt, die mit der ersten bzw. der zweiten Öffnung in Verbindung stehen, wobei die erste und die zweite Öffnung dazu ausgelegt sind, erste und zweite Schwenkzapfen der ersten und der zweiten Backenfähnchen (557a, 557b) durch die erste bzw. die zweite Mündung (1518) und in die erste bzw. die zweite Öffnung aufzunehmen.

13. Chirurgisches Ultraschallinstrument (110) nach Anspruch 12, ferner umfassend erste und zweite Füllstoffe (1520), die in Eingriff mit der ersten bzw. zweiten Mündung (1518) kommen, wodurch die erste und die zweite Öffnung umschlossen werden und der erste bzw. zweite Schwenkzapfen (559a, 559b) darin gehalten werden.

## Revendications

1. Instrument chirurgical à ultrasons (110), comprenant :
un ensemble effecteur terminal (500) comportant un corps retenant une lame à ultrasons (540) ;
la lame à ultrasons (540) s'étendant distalement à partir du corps (510) ;
un élément de mâchoire (550), comportant :
un cadre structurel rigide (552) comportant une partie proximale bifurquée (555) et une partie distale allongée (556), la partie proximale bifurquée (555) comportant des premier et second drapeaux de mâchoire (557a, 557b) accouplés pivotants au corps (510) pour permettre le pivotement de l'élément de mâchoire (550) par rapport à la lame à ultrasons (540) entre une position espacée et une position approchée ; et
une doublure de mâchoire souple (554) fixée à la partie distale allongée (556) du cadre structurel rigide et configurée pour faire face à la lame à ultrasons (540) dans la position approchée de l'élément de mâchoire ;
un câble d'ouverture de mâchoire (580) accouplé au premier drapeau de mâchoire (557a) au niveau d'un premier emplacement et acheminé autour de l'axe de pivot le long d'un premier trajet s'étendant autour d'une partie d'un bord externe du premier drapeau de mâchoire (557a) ; et
un câble de fermeture de mâchoire (590) accouplé au second drapeau de mâchoire (557b) au niveau d'un second emplacement et acheminé autour de l'axe de pivot le long d'un second trajet s'étendant autour d'une partie d'un bord externe du second drapeau de mâchoire (557b),
dans lequel les premier et second trajets sont respectivement configurés de telle sorte que la traction proximale du câble d'ouverture de mâchoire (580) fasse pivoter l'élément de mâchoire (550) vers la position espacée et de telle sorte que la traction proximale du câble de fermeture de mâchoire (590) fasse pivoter l'élément de mâchoire (550) vers la position approchée.

2. Instrument chirurgical à ultrasons (110) selon la revendication 1, comprenant en outre :
un transducteur à ultrasons (532) disposé à l'intérieur du corps (510) ; et
un pavillon à ultrasons (534) accouplant le transducteur à ultrasons (532) à la lame à ultrasons (540).

3. Instrument chirurgical à ultrasons (110) selon la revendication 2, dans lequel le pavillon à ultrasons (534) coopère avec le corps (510) pour enfermer de manière étanche le transducteur à ultrasons à l'intérieur d'un boîtier proximal du corps.

4. Instrument chirurgical à ultrasons (110) selon la revendication 3, dans lequel le pavillon à ultrasons (534) comporte une bride annulaire (537), et dans lequel la bride annulaire (537) est fixée entre un collier distal (514) du corps et le boîtier proximal du corps pour enfermer de manière étanche le transducteur à ultrasons (532) à l'intérieur du boîtier proximal du corps.

5. Instrument chirurgical à ultrasons (110) selon l'une quelconque des revendications 1 à 4, dans lequel les premier et second drapeaux de mâchoire (557a, 557b) comportent des premier et second bossages de pivot respectifs (559a, 559b) définissant un axe de pivot et configurés pour recevoir des première et seconde ouvertures de pivot (529), respectivement, définies à travers le corps sur des côtés opposés de la lame à ultrasons (540).

6. Instrument chirurgical à ultrasons (110) selon l'une quelconque des revendications 1 à 5, dans lequel :
le câble d'ouverture de mâchoire (580) comporte une première virole (582) en prise au niveau d'une extrémité distale de celle-ci, la première virole (582) étant capturée dans un premier évidement de capture du premier drapeau de mâchoire (557a) pour accoupler de ce fait le câble d'ouverture de mâchoire (580) au premier drapeau de mâchoire (557a) ; et
le câble de fermeture de mâchoire (590) comporte une seconde virole (592) en prise au niveau d'une extrémité distale de celle-ci, la seconde virole (592) étant capturée dans un second évidement de capture du second drapeau de mâchoire (557b) pour accoupler de ce fait le câble de fermeture de mâchoire (590) au second drapeau de mâchoire (557b).

7. Instrument chirurgical à ultrasons (110) selon la revendication 6, dans lequel les premier et second évidements de capture sont décalés différemment l'un de l'autre par rapport à un axe de pivot de l'élément de mâchoire.

8. Instrument chirurgical à ultrasons (110) selon l'une quelconque des revendications 1 à 7, dans lequel :
la partie du bord externe du premier drapeau de mâchoire (557a) définit un premier canal de guidage de câble (562a) configuré pour recevoir au moins partiellement le câble d'ouverture de mâchoire (580) ; et
la partie du bord externe du second drapeau de mâchoire (557b) définit un second canal de guidage de câble (562b) configuré pour recevoir au moins partiellement le câble de fermeture de mâchoire (590).

9. Instrument chirurgical à ultrasons (110) selon la revendication 8, dans lequel :
le corps (510) définit une première fente (527) et le premier canal de guidage configuré pour guider le câble d'ouverture de mâchoire (580) de manière proximale à partir de l'élément de mâchoire ; et
le corps définit une seconde fente (527) et le second canal de guidage configuré pour guider le câble de fermeture de mâchoire (590) de manière proximale à partir de l'élément de mâchoire.

10. Instrument chirurgical à ultrasons (110) selon l'une quelconque des revendications 1 à 9, dans lequel le corps (510) définit une extension proximale configurée pour accoupler le corps à une articulation.

11. Instrument chirurgical à ultrasons (110) selon l'une quelconque des revendications 1 à 10, dans lequel le corps (510) comporte une première partie de collier distal et une seconde partie de collier distal, les première et seconde parties de collier distal étant configurées pour entrer en prise l'une avec l'autre pour définir des première et seconde ouvertures de pivot configurées pour recevoir des premier et second bossages de pivot (559a, 559b) des premier et second drapeaux de mâchoire (557a, 557b), respectivement, pour accoupler de manière pivotante l'élément de mâchoire au corps.

12. Instrument chirurgical à ultrasons (110) selon l'une quelconque des revendications 1 à 11, dans lequel le corps (510) comporte un collier distal définissant des première et seconde ouvertures et comportant des première et seconde embouchures (1518) en communication avec les première et seconde ouvertures, respectivement, les première et seconde ouvertures étant configurées pour recevoir des premier et second bossages de pivot des premier et second drapeaux de mâchoire (557a, 557b) à travers les première et seconde embouchures (1518) et dans les première et seconde ouvertures, respectivement.

13. Instrument chirurgical à ultrasons (110) selon la revendication 12, comprenant en outre des première et seconde charges (1520) en prise dans les première et seconde embouchures (1518), respectivement, enfermant de ce fait les première et seconde ouvertures et capturant les premier et second bossages de pivot (559a, 559b), respectivement, dans celles-ci.
